(19)

Europäisches Patentamt

European Patent Office

Office européen des brevets

(11) **EP 4 361 882 A1**

(12) **EUROPEAN PATENT APPLICATION**
published in accordance with Art. 153(4) EPC

(43) Date of publication:
**01.05.2024 Bulletin 2024/18**

(21) Application number: **22827476.7**

(22) Date of filing: **17.06.2022**

(51) International Patent Classification (IPC):
**G06K 9/62** (2022.01)

(86) International application number:
**PCT/CN2022/099318**

(87) International publication number:
**WO 2022/267981 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.06.2021 CN 202110695472**

(71) Applicant: Ankon Technologies Co., Ltd
**Wuhan, Hubei 430000 (CN)**

(72) Inventors:
• **ZHANG, Hang**
  **Wuhan, Hubei 430040 (CN)**
• **GONG, Qiang**
  **Wuhan, Hubei 430040 (CN)**
• **YUAN, Wenjin**
  **Wuhan, Hubei 430040 (CN)**
• **ZHANG, Hao**
  **Wuhan, Hubei 430040 (CN)**

(74) Representative: **Bals & Vogel Patentanwälte PartGmbB**
**Konrad-Zuse-Str. 4**
**44801 Bochum (DE)**

(54) **ENDOSCOPIC IMAGE RECOGNITION METHOD, ELECTRONIC DEVICE, AND STORAGE MEDIUM**

(57) The present invention provides an endoscopic image recognition method, an electronic device, and a storage medium. The method includes: performing disease prediction for a plurality of disease categories for a plurality of original images respectively using a first neural network model; establishing test sample sets for the disease categories based on the disease prediction results for the original images, where each test sample set contains image features of a predefined number of original images; performing disease recognition for the test sample sets of the disease categories respectively using a second neural network model; superimposing the disease recognition results for the disease categories to obtain a case diagnosis result; where the second neural network model performs a weighted combination of a plurality of image features within the test sample sets to obtain the disease recognition results. The method improves the accuracy of disease recognition.

Perform disease prediction on an individual image from original images to obtain image features and a classification probability of the original images using a first neural network model — S01

For different disease categories, select image features from original images with the highest disease classification probabilities to form test sample sets — S02

Perform disease recognition on the test sample sets for the plurality of disease categories using a second neural network model — S03

Superimpose disease recognition results of the plurality of disease categories to obtain a case diagnosis result — S04

FIG. 3

EP 4 361 882 A1

**Description**

The application claims priority to Chinese Patent Application No. 202110695472.3, filed June 23, 2021, entitled " ENDOSCOPIC IMAGE RECOGNITION METHOD, ELECTRONIC DEVICE, AND STORAGE MEDIUM", all of which are incorporated herein by reference in their entirety.

**FIELD OF THE DISCLOSURE**

The present invention relates to the field of medical device imaging, and more particularly to an endoscopic image recognition method based on deep learning, an electronic device, and a storage medium.

**DESCRIPTION OF THE RELATED ART**

Capsule endoscope is an effective diagnostic and therapeutic tool for examining patients with gastrointestinal diseases. It incorporates devices such as a camera, LED lights, a wireless communication component. During the examination, the patient swallows a capsule endoscope, which travels through digestive tract, capturing images and transmitting the images outside the body of the patient. The captured images are then analyzed to identify lesions within the digestive tract. Compared to traditional endoscopy, the capsule endoscope offers the advantage of reduced patient discomfort and the ability to examine the entire digestive tract. This revolutionary technology has gained increasing recognition and application.

The capsule endoscope acquires a large number of images during the examination, e.g., tens of thousands of images. The process of image review and analysis has become challenging and time-consuming. With the development of technology, there is growing interest in utilizing image processing and computer vision techniques for lesion recognition. However, in existing endoscopic image recognition methods, each image captured by the capsule endoscope is individually subjected to lesion recognition using convolutional neural networks, obtaining diagnostic results. Even with a high recognition accuracy rate of up to 90%, an incorrect lesion recognition result for any single image among the numerous images captured in the digestive tract can lead to incorrect overall case diagnoses.

Therefore, there is still a demand for further improvements in endoscopic image recognition methods to improve the accuracy of case diagnoses based on a large number of images.

**SUMMARY OF THE DISCLOSURE**

In order to technically solve the above problems in the prior art, the present invention provides an endoscopic image recognition method, an electronic device, and a storage medium. After performing disease prediction on a plurality of original images based on a single image, disease recognition is performed for a plurality of image features of a test sample set based on the disease prediction results to improve the accuracy of disease recognition.

According to a first aspect of the present invention, an endoscopic image recognition method is provided, comprising: performing disease prediction for a plurality of disease categories for a plurality of original images respectively using a first neural network model; establishing test sample sets for the plurality of disease categories based on the disease prediction results for the plurality of original images, where each test sample set comprises image features of a predefined number of original images; performing disease recognition for the test sample sets of the plurality of disease categories respectively using a second neural network model; superimposing the disease recognition results for the plurality of disease categories to obtain a case diagnosis result; where the second neural network model performs a weighted combination of a plurality of image features within the test sample sets to obtain the disease recognition results.

Preferably, the first neural network model is a convolutional neural network model that takes individual images from the plurality of original images as input and outputs image features and classification probabilities for the plurality of disease categories.

Preferably, the second neural network model is a recurrent neural network model that takes the plurality of image features from the test sample sets as input and outputs the disease recognition results corresponding to the test sample sets.

Preferably, the second neural network model comprises: a first fully connected layer that individually performs dimensionality reductions on the plurality of image features from the test sample sets; a bidirectional long short-term memory layer that predicts hidden states for the dimension-reduced image features in a forward direction and a backward direction; and an attention mechanism that performs a weighted combination of the hidden states of the plurality of image features to obtain final features; where the second neural network model obtains the disease recognition results based on the final features.

Preferably, the first fully connected layer comprises a plurality of fully connected units, and the plurality of fully connected units separately perform dimensionality reductions on one corresponding image feature.

Preferably, the bidirectional long short-term memory layer comprises a plurality of forward long short-term memory units and a plurality of backward long short-term memory units. The plurality of forward long short-term memory units separately perform forward prediction for one corresponding image feature, and the plurality of backward long short-term memory units separately perform backward prediction for one corresponding image feature.

Preferably, the weighted combination comprises a weighted summation of the hidden states of the plurality of image features. Weight coefficients for the plurality of image features represent the influence on the disease recognition for the corresponding disease category.

Preferably, the weight coefficients for the plurality of image features are as shown in the formula below:

$$e_t = W_e \tanh(W_u H_t + b_u),$$

$$a_t = \mathrm{softmax}(e_t),$$

where, $W_u, W_e$ represents a weight matrix, $b_u$ represents a bias term, $H_t$ represents the hidden state obtained by the bidirectional long short-term memory layer in step t, $e_t$ represents an influence value, $a_t$ represents the weight coefficient.

Preferably, the step of "establishing test sample sets for the plurality of disease categories" comprises: for different disease categories within the plurality of disease categories, selecting the image features of a predefined number of original images with the highest classification probabilities from the plurality of original images to create the test sample sets.

Preferably, the predefined number is any integer within the range of 2-128.

Preferably, the plurality of original images are obtained using any of the following endoscopes: a fiberoptic endoscope, an active capsule endoscope, or a passive capsule endoscope.

According to a second aspect of the present invention, an electronic device is provided, comprising a memory and a processor, where the memory stores a computer program that can run on the processor, and the processor executes the program to implement the steps of the endoscopic image recognition method based on deep learning.

According to a third aspect of the present invention, a computer-readable storage medium is provided, on which a computer program is stored, and the computer program is executed by the processor to implement the steps of the endoscopic image recognition method based on deep learning.

In the embodiments of the present invention, a first neural network model is used for disease prediction and a second neural network model is used for disease recognition. In the second neural network, a weighted combination of a plurality of image features within the test sample sets is performed to obtain disease recognition results. As a result, the disease recognition accuracy is improved. Further, based on a plurality of test sample sets corresponding to a plurality of disease categories, a plurality of disease recognition results are obtained. The recognition results for the disease categories are superimposed to obtain the case diagnosis result.

In preferred embodiments, the second neural network model comprises a bidirectional long short-term memory layer which predicts the hidden states of a plurality of image features in a forward direction and a backward direction, and combines the image features from a previous moment and a subsequent moment to enhance the disease recognition accuracy further.

In preferred embodiments, each test sample set comprises image features from a predefined number of original images, such as 2-128 original images. Therefore, a balance between disease recognition accuracy and calculation time for disease categories can be achieved.

## BRIEF DESCRIPTION OF THE DRAWINGS

FIG. 1 illustrates a schematic structural diagram of a capsule endoscope system.

FIG.2 illustrates a schematic cross-sectional diagram of an example of a capsule endoscope.

FIG.3 illustrates a flow diagram of an endoscopic image recognition method according to an embodiment of the present invention

FIG.4 illustrates a schematic block diagram of the endoscopic image recognition method according to an embodiment of the present invention.

FIG. 5 illustrates a schematic block diagram of a first neural network model in the endoscopic image recognition method according to an embodiment of the present invention.

FIG.6 illustrates a schematic block diagram of a second neural network model in the endoscopic image recognition method according to an embodiment of the present invention.

## DETAILED DESCRIPTION OF EMBODIMENTS OF THE DISCLOSURE

The present invention can be described in detail below with reference to the accompanying drawings and preferred embodiments. However, the embodiments are not intended to limit the present invention, and the structural, method, or functional changes made by those skilled in the art in accordance with the embodiments are included in the scope of the present invention.

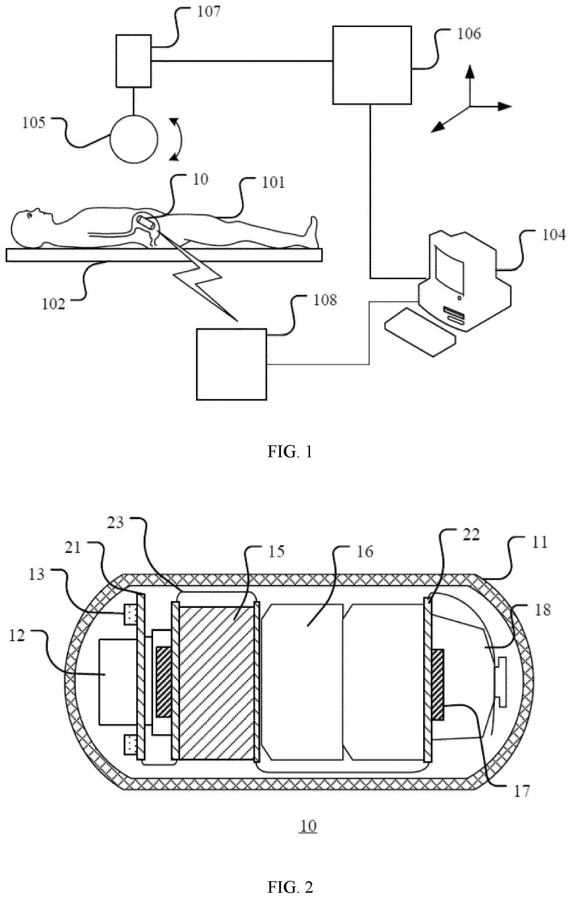

FIG.1 illustrates a schematic structural diagram of a capsule endoscope system. The capsule endoscope system, for example, comprises components such as a host 104, a magnetic ball 105, a three-axis movement base 106, a magnetic ball holder 107, and a wireless receiving device 108.

The magnetic ball holder 107 comprises a first end that connects to the three-axis movement base 106 and a second end that connects to the magnetic ball 105. The three-axis movement base 106, for example, is capable of translating along three coordinate axes perpendicular to each other. The magnetic ball holder 107 moves along with the three-axis movement base 106 and allows the magnetic ball 105 to rotate in the horizontal plane and the vertical plane relative to the magnetic ball holder 107. For example, the translation of the three-axis movement base 106 is driven by motors and screws, and the rotation of the magnetic ball 105 is driven by motors and belts. Therefore, the magnetic ball 105 can assume various orientations in five degrees of freedom. The magnetic ball 105, for example, comprises a permanent magnet which comprises opposing a North (N) pole and a South (S) pole. Changes in the orientation of the magnetic ball 105 lead to corresponding changes in the position and the orientation of an external magnetic field.

During an examination, a patient 101 swallows a capsule endoscope 10 while lying flat on a bed 102, for example. The capsule endoscope 10 travels along the digestive tract. As described below, the interior of the capsule endoscope 10 comprises a permanent magnet. The host 104 sends operational commands to the three-axis movement base 106 and the magnetic ball holder 107, thus controlling the orientation of the magnetic ball 105. The external magnetic field generated by the magnetic ball 105 acts on the permanent magnet, enabling control over the position and orientation of the capsule endoscope 10 within the digestive tract of the patient. While advancing through the digestive tract, the capsule endoscope 10 captures images and transmits the images to an external wireless receiving device 108. The host 104 is connected to the wireless receiving device 108, to acquire the images captured by the capsule endoscope 10, and facilitate analysis of the images for identification of lesions in the digestive tract.

FIG.2 illustrates a schematic cross-sectional diagram of an example of a capsule endoscope. The capsule endoscope 10 comprises an enclosure 11 and circuit components located inside the enclosure 11.

The enclosure 11 is composed of polymer materials such as plastic and comprises a transparent end to provide illumination and imaging pathways. The circuit components comprise an image sensor 12, a first printed circuit board (abbreviated as PCB) 21, a permanent magnet 15, a battery 16, a second PCB 22, and a wireless transmitter 17 arranged along the main axis of the enclosure 11. The image sensor 12 is positioned opposite the transparent end of the enclosure 11 and is, for example, mounted in the middle of the first PCB 21. On the first PCB 21 are further mounted a plurality of LEDs 13 surrounding the image sensor 12. The wireless transmitter 17 is mounted on the second PCB 22. The first PCB 21 is connected to the second PCB 22 via a flexible printed circuit 23, and the permanent magnet 15 and the battery 16 are held between the first PCB 21 and the second PCB 22. The flexible printed circuit 23 or an additional circuit board provides contact for the positive and negative terminals of the battery 16.

Further, the circuit components may further comprise a stop block 18 fixedly attached to the second PCB 22, used to securely clamp the flexible printed circuit 23 or to securely clamp the enclosure 11.

When the capsule endoscope 10 is capturing images, the LEDs 13 are lit, providing illumination through the transparent end of the enclosure, and the image sensor 12 captures images of the digestive tract through the transparent end of the enclosure. The image data is transmitted through the flexible printed circuit 23 to the wireless transmitter 17, which transmit the image data to the external wireless receiving device 108 outside the body of the patient, so that the host 104 can acquire the images for lesion analysis.

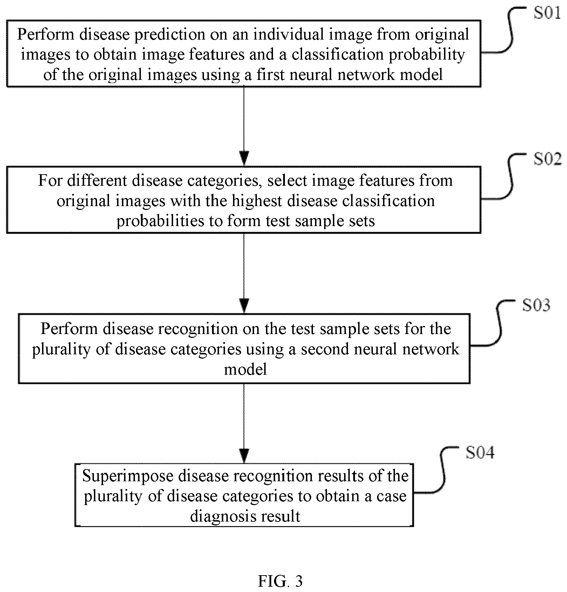

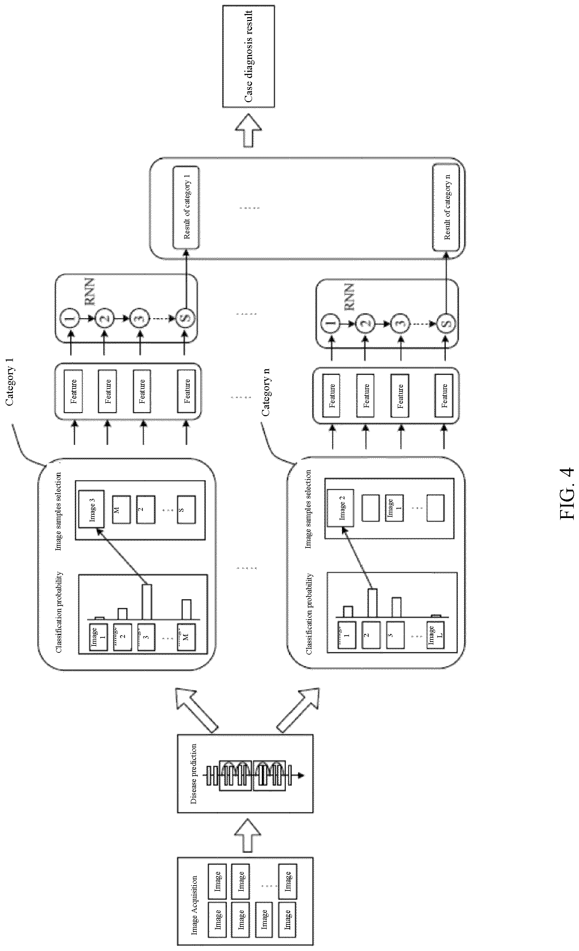

FIG.3 and FIG.4 illustrate a flow diagram and a schematic block diagram, respectively, of an endoscopic image recognition method according to an embodiment of the present invention.

In the capsule endoscope system as illustrated in FIG. 1, the position and the orientation of the capsule endoscope are controlled using the magnetic ball. The capsule endoscope acquires a large number of original images from various positions and orientations within the digestive tract of the patient. The host then executes the endoscopic image recognition method as illustrated in FIG. 3 to obtain case diagnosis results. The capsule endoscope system comprises an active capsule endoscope for capturing images of the digestive tract. This is just one way to obtain original images. In other embodiments, the original images can be images of the digestive tract obtained through a fiber-optic endoscope or captured by a passive capsule endoscope, among other methods.

In step S01, a first neural network model is used to perform disease prediction on an individual image from the original images in order to obtain image features and a classification probability for the disease categories of the original images, where the classification probability represents the probability of the individual image belonging to different disease categories. In the embodiment, the first neural network model, for example, is a convolutional neural network (abbreviated as CNN) model.

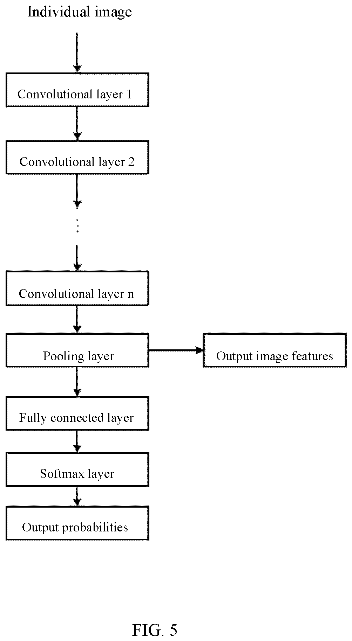

Referring to FIG. 5, the first neural network model comprises a plurality of convolutional layers, at least one pooling layer, at least one fully connected layer, and at least one normalized exponential layer (e.g., softmax layer). In the convolutional neural network model, convolution operations are used to extract different image features. The plurality of convolution layers sequentially extract low-level image features and high-level image features. The pooling layer downsamples the image features (i.e., low-level image features and high-level image features) to compress data and parameters of the image features while maintaining invariance of the image features. Each node of the fully connected layer is connected to all nodes from the previous layer and is used to synthesize the final features (i.e., the downsampled image features) extracted from the previous layer for classification. The normalized exponential layer maps the output from the previous layer (e.g., the fully connected layer) to probability values in the interval (0,1), thus obtaining the classification probabilities for the corresponding disease category. It can be understood that the first neural network model can be trained using annotated training sample sets. Individual images from the original images captured during the examination are used as the input for the first neutral network model, the image features are extracted from the pooling layer, and the classification probability is calculated from the normalized exponential layer.

The endoscopic image recognition method of the present invention is not limited to specific convolutional neural network (CNN) models. Commonly used network models like residual network (abbreviated as ResNet), densely connected convolutional networks (abbreviated as DenseNet), MobileNets, and others can also be used. For example, the applicant disclosed in Chinese patent application 202110010379.4 an example of a convolutional neural network model that can be used in this step. As described above, during the examination of the patient, the capsule endoscope can capture thousands of original images. The first neural network model takes individual images from the original images as input and processes each image to obtain a classification probability and a disease category corresponding to the image. The disease category comprises at least one of the following: erosion, bleeding, ulcer, polyp, protrusion, capillary dilation, vascular malformation, diverticulum, and parasites. In the embodiment, nine disease categories are listed, but it is understood that the number of disease categories recognized by the first neural network model can vary based on the training sample set. The present invention is not limited to specific number of disease categories.

In step S02, for different disease categories, image features are selected from a plurality of original images with the highest disease classification probabilities to form test sample sets.

For a plurality of disease categories, the plurality of original images for which disease prediction has been performed are sorted according to the classification probabilities. The image features of the original images with the highest classification probabilities for the respective disease categories are selected to form individual test sample sets. The image features in the test sample sets are preferably selected from the image features output by the pooling layer. The number of images S in the test sample sets for each disease category may be a predetermined number, for example, any integer within the range of 2 to 128, thereby balancing disease recognition accuracy and calculation time for disease category identification. In the embodiment, the number N of disease categories is 9 (N=9), and the number of images S in the test sample sets for each disease category is 10 (S=10). In other embodiments, the number of disease categories and the number of images in the test sample sets for each disease category can be adjusted as needed.

For example, referring to FIG. 4, captured images are input into the first neural network model (i.e. CNN model) for disease prediction. The first neural network model processes each captured image and, based on the image features of each image, obtains a probability that the captured image is identified as a different disease category. Accordingly, the captured images that are classified into category 1 include image 1, image 2, image 3, and so on up to image M, and image samples are selected in descending order of the classification probabilities, to obtain image 3, image M, image 2, and so on, up to image S. The processing for other categories is similar to category 1, so it is not further described here. Based on the selected image samples, the first neural network model outputs the image features corresponding to the image samples and forms a test sample set.

In step S03, a second neural network model is used to perform disease recognition on the test sample sets for the plurality of disease categories. The second neural network model, for example, is a recurrent neural network (abbreviated as RNN) model.

For each of the test sample sets for the plurality of disease categories, the second neural network model performs disease recognition based on the test sample sets of the image features extracted from the plurality of original images, i.e., based on the test sample sets output by the first neural network model, in order to enhance the accuracy of disease recognition. Referring to FIG. 4, for example, the first neural network model selects the S images with the highest probability of suspected erosion images, and takes the image features of a suspected category 1 (such as erosion) extracted from each of the S images as a test sample set. The test sample set is then input into the second neural network model, which can determine whether the disease of category 1 (e.g., erosion type disease) is actually present. The same process applies to other types of diseases.

In step S04, the disease recognition results of the plurality of disease categories are superimposed to obtain a case diagnosis result.

Following the aforementioned disease prediction and disease recognition steps, the large number of original images captured during the examination of the patient can be processed to obtain the recognition results of the plurality of disease categories, which are then superimposed to obtain the case diagnosis result. In a specific embodiment, the case diagnosis result is one or more of the nine disease categories included in the lesions of the patient. For example, for the above nine disease categories, if the recognition results of two disease categories of bleeding and polyps are that there are lesions, and the recognition results of other disease categories are that there are no lesions, then the case diagnosis result can be the superimposed all disease categories, that is, the patient has lesions of two disease categories: bleeding and polyps.

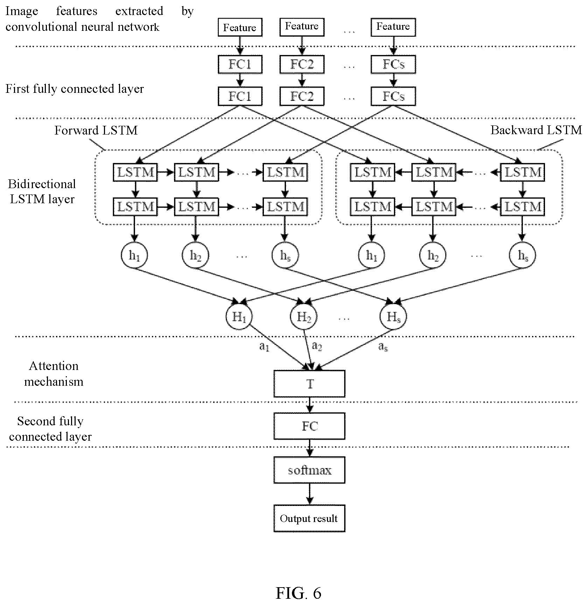

Referring to FIG.6, the second neural network model in the endoscopic image recognition method according to an embodiment of the present invention is described in detail below.

The second neural network model is a recurrent neural network (RNN) model. The recurrent neural network model refers to a recurrent neural network that takes sequential data as input. As shown in FIG.6, the second neural network model comprises at least one first fully connected layer, at least one bidirectional long short-term memory (abbreviated as LSTM) layer, an attention mechanism, at least one second fully connected layer, and at least one normalized exponential layer (e.g., a softmax layer).

The test sample sets of individual disease categories obtained from the disease predictions of the first neural network model are used as input to the second neural network model. The test sample set comprises a plurality of image features obtained from the plurality of original images.

The first fully connected layer comprises a plurality of fully connected units. The plurality of fully connected units separately perform dimensionality reductions on one corresponding image feature, i.e., the fully connected units respectively perform a dimensionality reduction on a plurality of image features in a high dimension to obtain a plurality of image features in a low dimension.

The bidirectional long short-term memory layer comprises a plurality of forward long short-term memory units and a plurality of backward long short-term memory units for predicting a hidden state for a plurality of image features in accordance with a forward direction and a backward direction, respectively. The plurality of forward long short-term memory units separately perform forward prediction for one corresponding image feature, and the plurality of backward long short-term memory units separately perform backward prediction for one corresponding image feature.

The inventors of the present invention have noticed that when doctors review and diagnose based on the images of the digestive tract (especially the images of the digestive tract continuously taken), they not only refer to images taken at the previous moment but also refer to images taken at the subsequent moment, and diagnose by combining the images at both the previous moment and the subsequent moment. The recurrent neural network model in existing capsule endoscope image processing methods uses a unidirectional long short-term memory layer, and thus can only predict the output of the next moment based on the input of the previous moment, instead of obtaining accurate disease recognition results based on the captured images. Unlike existing recurrent neural network model, the recurrent neural network model of the present invention uses a bidirectional long short-term memory layer to combine the image features of the previous moment and the subsequent moment for disease recognition.

In the bidirectional long short-term memory layer, the input of each forward long short-term memory unit is a corresponding image feature that has been dimension-reduced, and the output is a corresponding hidden state. The forward long short-term memory unit calculates the input image features in the order of input from front to back. The input of each backward long short-term memory unit is a corresponding image feature that has been dimension-reduced, and the output is a corresponding hidden state. The backward long short-term memory unit calculates the input image features in the order of input from back to front. The formula is as follows:

$$i_t = \sigma(W_{xi}x_t + W_{hi}h_{t-1} + b_i);$$

$$f_t = \sigma(W_{xf}x_t + W_{hf}h_{t-1} + b_f);$$

$$o_t = \sigma(W_{xo}x_t + W_{ho}h_{t-1} + b_o);$$

$$g_t = \sigma(W_{xg}x_t + W_{hg}h_{t-1} + b_g);$$

$$c_t = f_t \cdot c_{t-1} + i_t \cdot g_t;$$

$$h_t = o_t \cdot \varphi(c_t).$$

Where, $\varphi$ denotes the inverse tangent function, $\sigma$ denotes the sigmoid function, • denotes the dot product; $i, f, o, g$ denotes the four gates inside the long short-term memory unit; $x_t, h_{t-1}$ denotes the image feature and the hidden state respectively, and $c_t$ denotes the memory unit, with the subscripts t denoting the t-th step of the recursion, and t-1 denoting the t-1th step of the recursion; $W_{xi}, W_{xf}, W_{xo}, W_{xg}$ denotes the weights of the image feature x; $W_{hi}, W_{hf}, W_{ho}, W_{hg}$ denotes the weight matrix of the hidden state $h$; and $b_i, b_f, b_o, b_g$ denotes the bias term.

Further, the outputs of the forward long short-term memory unit and the backward long short-term memory unit corresponding to each image feature are superimposed to form their respective hidden states H, as shown in the following formula:

$$H_t = h_t + h_t'.$$

Where, $h_t$ denotes the hidden state of the forward long short-term memory unit at the t-th step, and $h_t'$ denotes the hidden state of the backward long short-term memory unit at the t-th step.

Therefore, the bidirectional long short-term memory layer can obtain a plurality of hidden states corresponding to the plurality of image features.

The attention mechanism of the second neural network model is used to perform a weighted combination of the hidden states of the plurality of image features to form a final feature.

Weight coefficients of each image feature represent the influence on disease recognition, as shown in the following formula:

$$e_t = W_e \tanh(W_u H_t + b_u),$$

$$a_t = \mathrm{softmax}(e_t).$$

Where, $W_u, W_e$ represents the weight matrix, $b_u$ represents the bias term, $H_t$ represents the hidden state obtained by the bidirectional long short-term memory layer in the step t, $e_t$ represents the influence value, $a_t$ represents the weight coefficient.

The hidden states of the plurality of image features are performed the weighted combination to form the final feature T, as shown in the following formula:

$$T = \sum_t a_t H_t.$$

Further, the second fully connected layer combines the final feature T extracted from the previous layer for classification. The normalized exponential layer maps the output from the previous layer (e.g., the second fully connected layer) to probability values in the interval $(0,1)$, thus obtaining the probability that each final feature T is classified into different disease categories, i.e., the probability of suspected disease category. Based on the probability of suspected disease category, the case diagnosis result is obtained and output.

The second neural network model performs disease recognition based on the test sample sets of image features from a plurality of original images, in order to determine whether the plurality of original images with the highest probability of suspected disease category actually contain lesions.

Further, an embodiment of the present invention provides an electronic device, comprising a memory and a processor. The memory stores a computer program that can run on the processor, and the processor executes the computer program to implement the steps of the endoscopic image recognition method based on deep learning.

Further, an embodiment of the present invention provides a computer-readable storage medium for storing a computer program. The computer program is executed by the processor to implement the steps of the endoscopic image recognition method based on deep learning.

In summary, according to the embodiments of the present invention, the deep learning-based endoscopic image recognition method, the electronic device, and the storage medium, after disease prediction on an individual image in the original images, a plurality of images are selected based on the disease prediction result and performed a weighted combination to improve the accuracy of disease recognition. The recognition results of a plurality of disease categories are superimposed to obtain the case diagnosis result.

For the convenience of description, the device is described in various modules divided by functions separately. When implementing the present invention, the functions of the various modules can be implemented in the same or different software and/or hardware.

The device implementations described above are merely illustrative. The modules described as separate components may or may not be physically separated, and the components displayed as modules may or may not be physical modules, that is, they may be located in one place, or may also be distributed over a plurality of network modules. Some or all of the modules may be selected according to actual needs to achieve the object of the embodiment. It can be understood and implemented by ordinary persons skilled in the art without creative work.

It should be understood that, although the description is described in terms of embodiments, not every embodiment merely comprises an independent technical solution. The description is recited in such a manner only for the sake of clarity, those skilled in the art should have the description as a whole, and the technical solutions in each embodiment may also be combined as appropriate to form other embodiments that can be understood by those skilled in the art.

The series of detailed descriptions set forth above are only specific descriptions of feasible embodiments of the present invention and are not intended to limit the scope of protection of the present invention. On the contrary, many modifications and variations are possible within the scope of the appended claims.

**Claims**

1. An endoscopic image recognition method, comprising:

    performing disease prediction for a plurality of disease categories for a plurality of original images respectively using a first neural network model;
    establishing test sample sets for the plurality of disease categories based on the disease prediction results for the plurality of original images, wherein each test sample set comprises image features of a predefined number of original images;
    performing disease recognition for the test sample sets of the plurality of disease categories respectively using a second neural network model; and

superimposing the disease recognition results for the plurality of disease categories to obtain a case diagnosis result;

wherein the second neural network model performs a weighted combination of a plurality of image features within the test sample sets to obtain the disease recognition results.

2. The endoscopic image recognition method of claim 1, wherein the first neural network model is a convolutional neural network model that takes individual images from the plurality of original images as input and outputs image features and classification probabilities for the plurality of disease categories.

3. The endoscopic image recognition method of claim 2, wherein the second neural network model is a recurrent neural network model that takes the plurality of image features from the test sample sets as input and outputs the disease recognition results corresponding to the test sample sets.

4. The endoscopic image recognition method of claim 1, wherein the second neural network model comprises:

a first fully connected layer that individually performs dimensionality reductions on the plurality of image features from the test sample sets;

a bidirectional long short-term memory layer that predicts hidden states for the dimension-reduced image features in a forward direction and a backward direction; and

an attention mechanism that performs a weighted combination of the hidden states of the plurality of image features to obtain final features;

wherein the second neural network model obtains the disease recognition results based on the final features.

5. The endoscopic image recognition method of claim 4, wherein the first fully connected layer comprises a plurality of fully connected units, and the plurality of fully connected units separately perform dimensionality reductions on one corresponding image feature.

6. The endoscopic image recognition method of claim 4, wherein the bidirectional long short-term memory layer comprises a plurality of forward long short-term memory units and a plurality of backward long short-term memory units, wherein the plurality of forward long short-term memory units separately perform forward prediction for one corresponding image feature, and the plurality of backward long short-term memory units separately perform backward prediction for one corresponding image feature.

7. The endoscopic image recognition method of claim 4, wherein the weighted combination comprises a weighted summation of the hidden states of the plurality of image features, wherein weight coefficients for the plurality of image features represent the influence on the disease recognition for the corresponding disease category.

8. The endoscopic image recognition method of claim 7, wherein the weight coefficients for the plurality of image features are as shown in the formula below:

$$e_t = W_e \tanh(W_u H_t + b_u),$$

$$a_t = \mathrm{softmax}(e_t),$$

wherein, $W_u$, $W_e$ represents a weight matrix, $b_u$ represents a bias term, $H_t$ represents the hidden state obtained by the bidirectional long short-term memory layer in step t, $e_t$ represents an influence value, $a_t$ represents the weight coefficient.

9. The endoscopic image recognition method of claim 2, wherein the step of "establishing test sample sets for the plurality of disease categories" comprises: for different disease categories within the plurality of disease categories, selecting the image features of a predefined number of original images with the highest classification probabilities from the plurality of original images to create the test sample sets.

10. The endoscopic image recognition method of claim 9, wherein the predefined number is any integer within the range of 2-128.

11. The endoscopic image recognition method of claim 1, wherein the plurality of original images are obtained using any of the following endoscopes: a fiber-optic endoscope, an active capsule endoscope, or a passive capsule endoscope.

12. An electronic device, comprising a memory and a processor, wherein the memory stores computer programs that run on the processor, and the processor executes the computer programs to implement the steps in the endoscopic image recognition method, wherein the method comprises:

performing disease prediction for a plurality of disease categories for a plurality of original images respectively using a first neural network model;

establishing test sample sets for the plurality of disease categories based on the disease prediction results for the plurality of original images, wherein each test sample set comprises image features of a predefined number of original images;

performing disease recognition for the test sample sets of the plurality of disease categories respectively using a second neural network model; and

superimposing the disease recognition results for the plurality of disease categories to obtain a case diagnosis result;

wherein the second neural network model performs a weighted combination of a plurality of image features within the test sample sets to obtain the disease recognition results.

13. A computer-readable storage medium having stored thereon a computer program, wherein the computer program is executed by the processor to implement the endoscopic image recognition method, wherein the method comprises:

performing disease prediction for a plurality of disease categories for a plurality of original images respectively using a first neural network model;

establishing test sample sets for the plurality of disease categories based on the disease prediction results for the plurality of original images, wherein each test sample set comprises image features of a predefined number of original images;

performing disease recognition for the test sample sets of the plurality of disease categories respectively using a second neural network model; and

superimposing the disease recognition results for the plurality of disease categories to obtain a case diagnosis result;

wherein the second neural network model performs a weighted combination of a plurality of image features within the test sample sets to obtain the disease recognition results.

FIG. 1

FIG. 2

Perform disease prediction on an individual image from original images to obtain image features and a classification probability of the original images using a first neural network model — S01

For different disease categories, select image features from original images with the highest disease classification probabilities to form test sample sets — S02

Perform disease recognition on the test sample sets for the plurality of disease categories using a second neural network model — S03

Superimpose disease recognition results of the plurality of disease categories to obtain a case diagnosis result — S04

FIG. 3

FIG. 4

Individual image

↓

Convolutional layer 1

↓

Convolutional layer 2

⋮

Convolutional layer n

↓

Pooling layer → Output image features

↓

Fully connected layer

↓

Softmax layer

↓

Output probabilities

FIG. 5

Image features extracted by convolutional neural network

First fully connected layer

Forward LSTM                                                                    Backward LSTM

Bidirectional LSTM layer

Attention mechanism

Second fully connected layer

FIG. 6

**INTERNATIONAL SEARCH REPORT**

| International application No. |
|---|
| **PCT/CN2022/099318** |

| A. | CLASSIFICATION OF SUBJECT MATTER |
|---|---|
| | G06K 9/62(2022.01)i |

According to International Patent Classification (IPC) or to both national classification and IPC

| B. | FIELDS SEARCHED |
|---|---|

Minimum documentation searched (classification system followed by classification symbols)

G06K

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 内窥镜, 影像, 图像, 图片, 预测, 分类, 测试, 模型, 叠加, 加权, 权重, 识别, 特征, endoscope, image, picture, prediction, classification, test, model, superposition, weight, feature

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 113159238 A (ANKON TECHNOLOGIES (WUHAN) CO., LTD.) 23 July 2021 (2021-07-23) <br> claims 1-13 | 1-13 |
| A | CN 112348125 A (ANKON TECHNOLOGIES (WUHAN) CO., LTD.) 09 February 2021 (2021-02-09) <br> description, paragraphs [0007] to [0015] | 1-13 |
| A | CN 111539930 A (ZHEJIANG DEMETICS MEDICAL TECHNOLOGY CO., LTD.) 14 August 2020 (2020-08-14) <br> entire document | 1-13 |
| A | CN 111275118 A (FUDAN UNIVERSITY) 12 June 2020 (2020-06-12) <br> entire document | 1-13 |
| A | US 2020381083 A1 (410 AI, LLC.) 03 December 2020 (2020-12-03) <br> entire document | 1-13 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 September 2022** | **14 September 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2022/099318**

| Patent document cited in search report | | | Publication date (day/month/year) | Patent family member(s) | | | Publication date (day/month/year) |
|---|---|---|---|---|---|---|---|
| CN | 113159238 | A | 23 July 2021 | None | | | |
| CN | 112348125 | A | 09 February 2021 | None | | | |
| CN | 111539930 | A | 14 August 2020 | None | | | |
| CN | 111275118 | A | 12 June 2020 | None | | | |
| US | 2020381083 | A1 | 03 December 2020 | WO | 2020243526 | A1 | 03 December 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110695472
- CN 202110010379